# EUROPÄISCHE PATENTSCHRIFT

(11) **EP 2 507 385 B1**
(45) Veröffentlichungstag und Bekanntmachung des Hinweises auf die Patenterteilung: **11.02.2015**
(21) Anmeldenummer: 10784315.3
(22) Anmeldetag: 01.12.2010
(51) Int. Cl.: C12Q 1/68

(54) **VERFAHREN ZUM NACHWEIS UND ZUR QUANTIFIZIERUNG VON POLY(A)RNA UND mRNA**
METHOD FOR DETECTING AND QUANTIFYING POLY(A) RNA AND MRNA
PROCÉDÉ POUR LA DÉTECTION ET POUR LA QUANTIFICATION D'ARN POLY(A) ET D'ARNM

(30) Priorität: 04.12.2009 DE 102009056729
(43) Veröffentlichungstag der Anmeldung: 10.10.2012
(73) Patentinhaber: Qiagen GmbH, 40724 Hilden (DE)
(72) Erfinder: FANG, Nan, 40724 Hilden (DE)
(86) Internationale Anmeldenummer: PCT/EP2010/068663
(87) Internationale Veröffentlichungsnummer: WO 2011/067299

(56) Entgegenhaltungen:
- US-A- 5 976 797
- US-A1- 2004 076 994
- WONG MARISA L ET AL: "Real-time PCR for mRNA quantitation", BIOTECHNIQUES, INFORMA LIFE SCIENCES PUBLISHING, WESTBOROUGH, MA, US, Bd. 39, Nr. 1, 1. Juli 2005 (2005-07-01), Seiten 75-85, XP002556339, ISSN: 0736-6205, DOI: DOI:10.2144/05391RV01

## Beschreibung

Die vorliegende Erfindung betrifft ein Verfahren zum Nachweis und/oder zur Quantifizierung von Poly(A)RNA und/oder mRNA, wobei ein Poly(dT)-Oligonukleotid, welches einen Fluoreszenzfarbstoff sowie einen Quencher aufweist, an die nachzuweisende Nukleinsäure hybridisiert wird. Nicht hybridisiertes Poly(dT)-Oligonukleotid wird durch eine hinzugefügte Nuklease abgebaut, wodurch Fluoreszenz markierte Nukleotide freigesetzt werden und das entstandene Fluoreszenzsignal gemessen wird.

Die Quantifizierung der genauen Menge an eingesetzter mRNA ist für viele molekularbiologische Verfahren von großer Bedeutung. Insbesondere für verschiedene Genexpressions-Analysen wie beispielsweise der quantitiativen reversen Transkriptions-PCR (qRT-PCR) und Microarrays ist diese so genannte Normalisierung wichtig, um zu exakten und vergleichbaren Versuchsergebnissen zu gelangen.
Die eingesetzte Menge an RNA wird üblicherweise durch Spektrophotometrie durch Messen der Absorption bei 260 nm bestimmt. Diese Methode hat jedoch den Nachteil, dass bei dieser Wellenlänge nicht nur die mRNA sondern auch weitere Nukleinsäuren wie DNA, rRNA, tRNA und weitere nicht kodierende RNAs nachgewiesen werden, die exakte Menge an vorhandener mRNA also nicht bekannt ist.
Aus diesem Grund wird im Allgemeinen vor der Quantifizierung der RNA die DNA durch ein Aufreinigungsverfahren aus der Probe entfernt, was zusätzliche Arbeitsschritte und Kosten bedeutet. Nach dem Entfernen der DNA wird im Spektrophotometer die Menge an Gesamt-RNA bestimmt und daraus indirekt auf die Menge an vorhandener mRNA geschlossen. Da aber das Verhältnis von mRNA zu Gesamt-RNA nicht konstant ist, sondern von Präparation zu Präparation sowie bei verschiedenen Ausgangsmaterialien völlig unterschiedlich sein kann, ergibt sich eine hohe Fehlerrate, wenn von der Gesamt-RNA auf die Menge der vorhandenen mRNA geschlossen wird.
Ein weiterer Nachteil dieser Methode ist, dass nicht festgestellt werden kann, ob die mRNA in der Probe bereits (teilweise) abgebaut worden ist. Der Abbau von mRNAs beginnt häufig mit dem Entfernen des Poly(A)-Schwanzes am 3'-Ende und setzt sich mit dem Abbau des kodierenden Bereiches fort. Solche mRNA ist nicht mehr intakt und häufig nicht mehr für molekularbiologische Experimente verwendbar.

Um intakte, quantifizierbare Gesamt-mRNA in eine molekularbiologische Reaktion einsetzen zu können, muss diese zuvor aus der biologischen Probe in einem zeitaufwendigen und mehrschrittigen Verfahren mit Hilfe von Poly(dT)-Nukleotiden, die an eine feste Phase gekoppelt sind, aufgereinigt werden. Dieses Verfahren ist aber nicht quantitativ, so dass während der Aufreinigungsprozedur ein Teil der mRNA aus der biologischen Probe verloren geht. Nach der Aufreinigung muss die so isolierte mRNA spektrophotometrisch quantifiziert werden, was nicht nur einen zusätzlichen Arbeitsschritt sondern auch einen Verlust eines Teils der isolierten mRNA bedeutet.

US 2004/076994 (YAKU HIDENOBU [JP] ET AL) beschreibt den Nachweis einer Zielnukleinsäure mit Hilfe einer Nukleinsäuresonde, welche mit einem Fluoreszenz-Donor und einem Fluoreszenz-Akzeptor markiert ist. Nach Zusatz einer Nuklease wird Zielmolekülabhängige Veränderung der Fluoreszenz gemessen. US 5 976 797 (MITSUHASHI MASATO [US]) beschreibt die Bestimmung der Gesamt-mRNA Menge mit Hilfe von immobilisierten oligo-(dT) Sonden.

Aufgabe der vorliegenden Erfindung ist es, die Nachteile der aus dem Stand der Technik bekannten Verfahren zu überwinden und ein kostengünstiges und wenig zeitaufwendiges Verfahren zum spezifischen Nachweis und/oder zur Quantifizierung von mRNA und Poly(A)RNA zur Verfügung zu stellen, bei dem zuvor die Gesamt-RNA nicht von der mRNA-Fraktion abgetrennt werden muss.

Diese Aufgabe wird gelöst durch ein Verfahren gemäß Anspruch 1 zum spezifischen Nachweis und/oder zur Quantifizierung von Poly(A)RNA und/oder mRNA, aufweisend die folgenden Verfahrensschritte:
i) In Kontakt Bringen einer Poly(A)RNA- und/oder mRNA-haltigen Probe mit einem Poly(dT)-Oligonukleotid, welches einen Fluoreszenzfarbstoff sowie einen Quencher aufweist, wobei der Quencher bei der emittierenden Wellenlänge des Fluoreszenzfarbstoffes die Fluoreszenz im Poly(dT)-Oligonukleotid sowohl im hybridisierten als auch im nicht hybridisierten Zustand signifikant und in gleichem Maße löscht;
ii) Hybridisieren des Poly(dT)-Oligonukleotids an die Poly(A)RNA und/oder mRNA, wobei das Fluoreszenz-Signal unverändert bleibt;
iii) In Kontakt Bringen des Hybridisierungsansatzes aus Schritt ii) mit einer Einzelstrang-spezifischen Nuklease, wobei Fluoreszenz-markierte Nukleotide aus denjenigen Poly(dT)-Oligonukleotiden freigesetzt werden, die nicht mit Poly(A)RNA und/oder mRNA hybridisiert sind, wodurch das Fluoreszenzsignal erhöht wird;
iv) Messen des entstandenen Fluoreszenzsignals.

Das erfindungsgemäße Verfahren ist geeignet, alle Ribonukleinsäuren spezifisch nachzuweisen und zu quantifizieren, die einen Bereich aufweisen, der aus etwa 10 oder mehr aufeinanderfolgenden Adenin-Ribonukleotiden besteht. Zu diesen Ribonukleinsäuren gehören in erster Linie natürlich vorkommende messenger Ribonukleinsäuren (mRNAs), die im Allgemeinen an ihrem 3'-Ende einen so genannten Poly(A)-Schwanz aufweisen, der aus etwa 10 bis zu mehreren hundert aufeinanderfolgenden Adenin-Ribonukleotiden besteht.
Dadurch, dass der Abbau von mRNAs häufig mit dem Entfernen des Poly(A)-Schwanzes am 3'-Ende beginnt, wird durch das erfindungsgemäße Verfahren nur intakte, nicht degradierte und damit in molekularbiologischen Reaktionen voll funktionsfähige mRNA nachgewiesen und quantifiziert.
Desweiteren lassen sich mit dem erfindungsgemäßen Verfahren auch künstliche Ribonukleinsäuren, die an einem Ende oder innerhalb ihrer Sequenz einen Bereich von etwa 10 oder mehr aufeinanderfolgenden Adenin-Ribonukleotiden aufweisen (Poly(A)RNA), nachweisen und quantifizieren. Diese künstlichen Ribonukleinsäuren lassen sich beispielsweise als interner Standard für die Quantifizierung von mRNAs verwenden, wenn sie in vorher bekannter Konzentration eingesetzt werden.
Werden im erfindungsgemäßen Verfahren künstliche Ribonukleinsäuren, die einen Poly(A)-Bereich aufweisen oder natürliche mRNAs, deren Konzentration bekannt ist, in parallelen Ansätzen in unterschiedlichen Konzentrationen eingesetzt, so ist die entstandene Fluoreszenz abhängig von der eingesetzten Menge an RNA. Je höher die Menge an eingesetzter RNA ist, desto geringer ist die Fluoreszenz, die detektiert werden kann. Durch Korrelation der bekannten eingesetzten Menge an RNA mit der resultierenden Fluoreszenz lässt sich eine Eichkurve erstellen. Mit Hilfe dieser Eichkurve ist es möglich, in einem Ansatz, in dem die Menge an mRNA und/oder Poly(A)-RNA unbekannt ist, diese zu bestimmen, indem die detektierte Fluoreszenz in diesem Ansatz mit Hilfe der Eichgeraden einer RNA-Menge zugeordnet wird.

Das Poly(dT)-Oligonukleotid, welches zum Nachweis und zur Quantifizierung von mRNAs und Poly(A)RNA verwendet werden kann, weist sowohl einen Fluoreszenzfarbstoff als auch einen Quencher auf.
Unter einem Fluoreszenzfarbstoff wird im Sinne der vorliegenden Erfindung ein Farbstoff verstanden, der nach Anregung bei einer bestimmten Wellenlänge Licht einer größeren Wellenlänge emittiert.
Unter einem Quencher wird im Sinne der vorliegenden Erfindung ein Molekül verstanden, welches in der Lage ist, durch Energietransfer von einem Fluoreszenzmolekül in einen angeregten Zustand überzugehen und die Energie anschließend strahlungslos wieder abzugeben.
Dabei ist die Art des Quenchers so gewählt, dass er in der Lage ist, bei der emittierenden Wellenlänge des Fluoreszenzfarbstoffes die Fluoreszenz im Poly(dT)-Oligonukleotid in dem Maße zu löschen, dass eine Freisetzung des Fluoreszenzfarbstoffes aus dem Poly(dT)-Oligonukleotid zu einer detektierbaren Fluoreszenzerhöhung führt. Der Quencher löscht im erfindungsgemäßen Verfahren die Fluoreszenz des Fluoreszenzfarbstoffes im gleichen Maße unabhängig davon, ob das Poly(dT)-Oligonukleotid als Einzelstrang frei vorliegt oder mit einer weiteren Nukleinsäure hybridisiert vorliegt. Das Poly(dT)-Oligonukleotid bildet im freien, nicht an eine weitere Nukleinsäure gebundenen Zustand, keine Sekundärstruktur aus, die einen Einfluss auf den Abstand zwischen Fluoreszenzfarbstoff und Quencher hat. Solange also der Fluoreszenzfarbstoff und der Quencher im Poly(dT)-Oligonukleotid vorhanden sind, bleibt die Fluoreszenz auf demselben Niveau.
Der Abstand zwischen Fluoreszenzfarbstoff und Quencher im Poly(dT)-Oligonukleotid ist so gewählt, dass der Quencher in der Lage ist, die Fluoreszenz des Fluoreszenzfarbstoffes in dem Maße zu löschen, dass eine Freisetzung des Fluoreszenzfarbstoffes aus dem Poly(dT)-Oligonukleotid zu einer detektierbaren Fluoreszenzerhöhung führt.

In einer bevorzugten Ausführungsform löscht der Quencher die Fluoreszenz des Fluoreszenzfarbstoffes vollständig.

Das Poly(dT)-Oligonukleotid im erfindungsgemäßen Verfahren weist in einer bevorzugten Ausführungsform eine Länge von 10 bis 100 Nukleotiden auf.
In einer besonders bevorzugten Ausführungsform weist das Poly(dT)-Oligonukleotid eine Länge von 18 bis 25 Nukleotiden auf.
Das erfindungsgemäße Poly(dT)-Oligonukleotid kann ausschließlich aus (dT)-Nukleotiden aufgebaut sein. Demzufolge ergibt sich bei der Hybridisierung des Poly(dT)-Oligonukleotids an eine Poly(A)RNA oder eine mRNA keine exakte Positionierung des Poly(dT)-Oligonukeotids an diese RNAs, sofern die Länge der Poly(A)-Nukleotide in diesen RNAs länger ist als das Poly(dT)-Oligonukleotid. Ist die Länge der Poly(A)-Nukleotide mehr als doppelt so lang wie die Länge des Poly(dT)-Oligonukleotids, so kann mehr als ein Poly(dT)-Oligonukleotid an diese Poly(A)-Nukleotide der nachzuweisenden und zu quantifizierenden RNAs binden.

In einer weiteren Ausführungsform ist das Poly(dT)-Oligonukleotid eine Mischung aus unterschiedlichen Poly(dT)-Oligonukleotiden, die an ihrem 3'-Ende mindestens ein weiteres Nukleotid aufweisen, welches nicht ein (dT)-Nukleotid ist. Dieses mindestens eine nicht (dT)-Nukleotid am 3'-Ende fungiert als so genannter Anker, der dafür sorgt, dass das Poly(dT)-Oligonukleotid nicht an einer beliebigen Stelle des Poly(A)-Bereiches der Ribonukleinsäure hybridisieren kann sondern genau am Übergangsbereich zwischen der übrigen Sequenz der Ribonukleinsäure und dem sich 3' davon anschließenden Poly(A)-Bereich hybridisiert. Dabei werden die Hybridisierungsbedingungen in Schritt ii) des erfindungsgemäßen Verfahrens so eingestellt, dass das Poly(dT)-Oligonukleotid mit seinem Ankerbereich nur am Übergangbereich der Ribonukleinsäure hybridisieren kann, so dass nur genau ein Poly(dT)-Oligonukleotid an die Ribonukleinsäure hybridisieren kann, unabhängig davon, wie lang der Poly(A)-Bereich in diesem Molekül ist. Dem Fachmann sind die Einflussfaktoren geläufig, die es ermöglichen, die Hybridisierungsbedingungen entsprechend einzustellen.
Der Anker-Bereich ist dabei nicht bei allen verwendeten Poly(dT)-Oligonukleotiden in seiner Nukleotidsequenz identisch sondern ist eine Mischung, die so gewählt ist, dass gewährleistet ist, dass bei der Hybridisierung in Schritt ii) des erfindungsgemäßen Verfahrens an alle in der Probe vorhandenen mRNAs in deren Übergangsbereich zwischen Poly(A)-Bereich und ihrer übrigen Nukleotidsequenz genau ein Poly(dT)-Oligonukleotid hybridisieren kann. Dabei kann es ausreichen, dass der Ankerbereich lediglich ein einziges Nukleotid aufweist, er kann aber auch zwei oder mehr Nukleotide aufweisen.

In einer bevorzugten Ausführungsform beträgt der Abstand zwischen Fluoreszenzfarbstoff und Quencher im Poly(dT)-Oligonukleotid maximal 30 Nukleotide. Dabei können sowohl Fluoreszenzfarbstoff als auch Quencher an endständigen Nukleotiden als auch an innerhalb des Poly(dT)-Oligonukleotids liegenden Nukleotiden angebracht sein.
In einer besonders bevorzugten Ausführungsform sind Fluoreszenzfarbstoff und Quencher im Poly(dT)-Oligonukleotid am gegenüberliegenden Ende des Oligonukleotids anbebracht. Dabei spielt es im erfindungsgemäßen Verfahren keine Rolle, welches der beiden Moleküle am 5'-Ende und welches am 3'-Ende angebracht ist.

In einer bevorzugten Ausführungsform ist der Fluoreszenzfarbstoff ausgewählt aus der Gruppe 6-FAM, 6-JOE, Alexa Fluor 568, Alexa Fluor 633, Alexa Fluor 680, Bodipy, CAL Fluor, CAL Fluor Red 610, TAMRA, HEX, Oregon Green, TET, Texas Red, Marina Blue, Edans Bothell Blue, Fluorescein, Yakima Yellow, Glod 540, Cy3.5 und Cy5.

In einer bevorzugten Ausführungsform ist der Quencher ausgewählt aus der Gruppe DABCYL, BHQ21, BHQ2 und TAMRA.

In Schritt ii) des erfindungsgemäßen Verfahrens hybridisiert das Poly(dT)-Oligonukleotid an die Poly(A)RNA und/oder die mRNA, die in der Probe vorhanden sind. Da das Poly(dT)-Oligonukleotid zwangsläufig keine Sekundärstruktur ausbildet, ändert sich der Abstand zwischen Fluoreszenzfarbstoff und Quencher nicht, wenn das Poly(dT)-Oligonukleotid an die Ziel-RNA hybridisiert, die Fluoreszenz bleibt also vor, während und nach dem Hybridisierungsschritt des erfindungsgemäßen Verfahrens auf dem gleichen konstanten (niedrigen) Niveau. Wenn der Quencher so gewählt ist, dass er die Fluoreszenz des Fluoreszenzfarbstoffes vollständig löscht, ist sowohl nach Schritt i), in der das Poly(dT)-Oligonukleotid noch im nicht hybridisierten Zustand vorliegt, als auch nach Schritt ii), nachdem das Poly(dT)-Oligonukleotid an die Ziel-RNA gebunden hat, keine Fluoreszenz detektierbar.

Nachdem in Schritt ii) des erfindungsgemäßen Verfahrens das Poly(dT)-Oligonukleotid an die Poly(A)RNA und/oder die mRNA gebunden hat, wird in Schritt iii) eine Einzelstrang-spezifische Nuklease mit diesem Hybridisierungsansatz in Kontakt gebracht. Die Nuklease kann bereits während des Hybridisierungsschrittes im Hybridisierungsansatz vorhanden sein, sie kann aber auch erst dann mit dem Hybridisierungsansatz in Kontakt gebracht werden, nachdem die Hybridisierung abgeschlossen ist.
Als Einzelstrang spezifische Nuklease können alle Nukleasen verwendet werden, die in der Lage sind, einzelsträngige Nukleinsäuren zu kürzeren Oligonukleotiden oder zu ihren Nukleotiden abzubauen, doppelsträngige Nukleinsäuren jedoch intakt lassen. Geeignete Nukleasen sind Exonukleasen, die die einzelsträngige Nukleinsäure entweder von ihrem 3'-Ende (3' → 5' Exonuklease) oder von ihrem 5'-Ende (5' → 3' Exonuklease) beginnend abbauen, sowie Endonukleasen.

In einer bevorzugten Ausführungsform ist die Einzelstrang-spezifische Nuklease ausgewählt aus der Gruppe Exonuklease I, S1 Nuklease, Mung Bean Nuclease, Exonuklease T und RecJ oder eine Mischung von diesen.
In einer besonders bevorzugten Ausführungsform ist die Einzelstrang-spezifische Nuklease eine Exonuklease, ganz besonders bevorzugt Exonuklease I oder Exonuklease T oder eine Mischung von diesen.
Die Einzelstrang-spezifische Nuklease baut während dieses Schrittes alle frei vorliegenden, nicht hybridisierten Poly(dT)-Oligonukleotide zu kürzeren Oligonukleotiden oder zu ihren Einzelnukleotiden ab. Da die Nuklease Einzelstrang-spezifisch ist, werden Poly(dT)-Oligonukleotide, die mit einer Poly(A)RNA und/oder mRNA hybridisiert vorliegen, nicht von dieser Nuklease erkannt und folglich auch nicht zu kürzeren Oligonukleotiden oder zu ihren Nukleotiden abgebaut.
Durch den Abbau der nicht hybridisierten Poly(dT)-Oligonukleotide zu kürzeren Oligonukleotiden oder zu ihren Einzelnukleotiden liegen nach Schritt iii) auch die Nukleotide als kürzere Oligonukleotide oder als Einzelnukleotide vor, die einen Fluoreszenzfarbstoff bzw. einen Quencher aufweisen. Die räumliche Nähe zwischen Quencher und Fluoreszenzfarbstoff ist nun nicht mehr gegeben, so dass der Quencher die Emission des Fluoreszenzfarbstoffes nicht mehr löschen kann, wodurch sich die Fluoreszenz messbar erhöht.
Die Erhöhung des Fluoreszenzsignals wird in Schritt iv) gemessen. Zur Messung des Fluoreszenzsignals eignen sich alle Analysegeräte, die in der Lage sind, Licht der Anregungswellenlänge des Fluoreszenzfarbstoffes zu emittieren sowie Licht der Emissionswellenlänge des Fluoreszenzfarbstoffes zu detektieren. Zu diesen Analysegeräten zählen beispielsweise Spektrophotometer, Real-Time PCR-Cycler und Fluoreszenzmikroskope. Weitere geeignete Analysegeräte sind dem Fachmann geläufig.
Das entstandene Fluoreszenzsignal ist umso höher, je mehr nicht hybridisiertes Poly(dT)-Oligonukleotid im Reaktionsansatz vorliegt. Wird bei unterschiedlichen Proben die gleiche Menge an Poly(dT)-Oligonukleotid in das erfindungsgemäße Verfahren eingesetzt, so ist das entstandene Fluoreszenzsignal in der Probe höher, in der weniger Poly(A)-RNA und/oder mRNA vorhanden gewesen sind, da in dieser mehr nicht hybridisiertes Poly(dT)-Oligonukleotid vorliegt, so dass die Proben relativ zueinander quantifiziert werden können.
Da das entstandene Fluoreszenzsignal umso höher ist, je mehr Poly(dT)-Oligonukleotid nicht hybridisiert, eignet sich das erfindungsgemäße Verfahren nicht nur zum spezifischen Nachweis von Poly(A)RNA und mRNA, sondern auch für deren relative (siehe oben) und absolute (siehe unten) Quantifizierung.
Durch Verwenden eines internen Standards aus künstlicher Poly(A)RNA oder aus natürlich vorkommender mRNA, deren Menge bekannt ist, lässt sich eine Eichkurve aus Konzentration an RNA in Relation zur entstandenen Fluoreszenz bei einer konstanten Menge an Poly(dT)-Oligonukleotid erstellen. Durch Vergleich der Fluoreszenz, die in einer unbekannten Probe gemessen wird, mit der entsprechen Fluoreszenz in der Eichkurve ist es möglich, die absolute Konzentration an mRNA und/oder Poly(A)-RNA in dieser unbekannten Probe zu bestimmen.

Das erfindungsgemäße Verfahren eignet sich hervorragend für den schnellen und einfachen Nachweis sowie für die Quantifizierung von Poly(A)RNA und mRNA. Dieses Verfahren ist insbesondere hilfreich zur Normalisierung von Daten bei Genexpressionsanalysen wie beispielsweise der quantitativen RT-PCR, bei Microarray-Anwendungen und Northern-Blots, um bei diesen Verfahren die genaue Menge an eingesetzter mRNA bestimmen zu können. Weitere Einsatzmöglichkeiten des erfindungsgemäßen Verfahrens sind dem Fachmann geläufig.

### Abbildungen:

### Abbildung 1:

Kinetische Darstellung des Fluoreszenzsignals während des Exonuklease I-Verdaus bei unterschiedlichen Gesamt-RNA Mengen. Auf der X-Achse ist die Zeit (in Minuten), auf der Y-Achse die Fluoreszenz angegeben.

### Abbildung 2:

Lineares Verhältnis zwischen eingesetzter Menge an Gesamt-RNA und dem entstandenen Fluoreszenzsignal über einen zeitlichen Verlauf. Auf der X-Achse ist die Menge an eingesetzer RNA (in µg) angegeben, auf der Y-Achse ist das Fluoreszenzsignal als Differenz der gemessenen Fluoreszenz ohne RNA (maximale Fluoreszenz) und der gemessenen Fluoreszenz bei der Probe aufgetragen.

### Beispiele:

Das nachfolgende Beispiel ist dazu gedacht, die Erfindung weiter zu erläutern, ohne dass diese auf das Ausführungsbeispiel beschränkt sein soll.

### Beispiel 1:

Nachweis und Quantifizierung von RNA mit Poly(dT)-Oligonukleotiden Unterschiedliche Mengen an Gesamt-RNA (0 µg, 0,5 µg, 1 µg, 2 µg), die zuvor aus der menschlichen Zellkultur-Linie HeLa isoliert worden war, wurden mit 0,2 µM eines 22 Nukleotide langen Poly(dT)-Oligonukleotids, das am 5'-Ende mit FAM und am 3'-Ende mit TAMRA markiert war, hybridisiert. Die Hybridisierungsansatz wurde bei Raumtemperatur in einem Gesamtvolumen von 20 µl in OmniScript RT Puffer (QIAGEN) angesetzt. Unmittelbar anschließend wurden 20 U Exonuklease I (Epicentre) zur Hybridisierungsreaktion auf Eis hinzugefügt und die Exonuklease-Reaktion bei 25 °C in einem Rotorgene 6000 Real-Time PCR Cycler (QIAGEN) für 60 min durchgeführt. Dabei wurde jede Minute das entstandene Fluoreszenzsignal bei einer Anregungswellenlänge von 470 nm und einer Detektionswellenlänge von 510 nm gemessen.

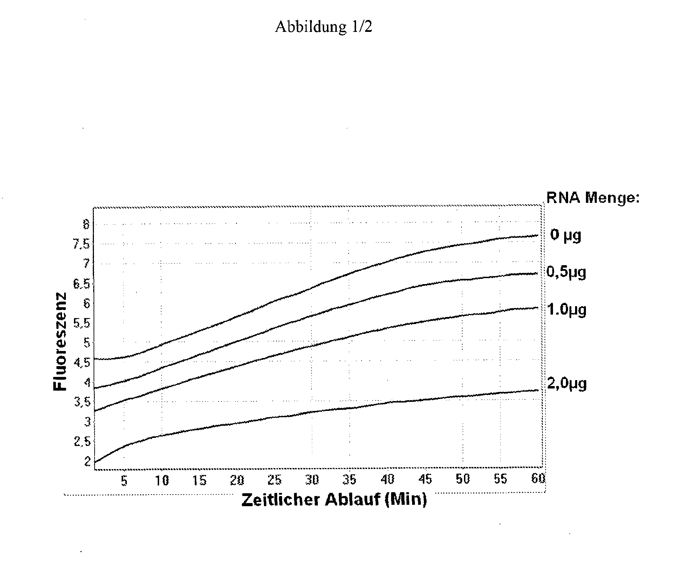

In Abbildung 1 ist der kinetische Verlauf der Fluoreszenzsignale während des Exonuklease I-Verdaus in Abhängigkeit der eingesetzten Gesamt-RNA Menge wiedergegeben.
Dabei zeigte sich, dass die Probe, in die keine RNA eingesetzt wurde, das höchste Fluoreszenzsignal generierte, da in dieser Probe die höchste Menge an nicht hybridisiertem Poly(dT)-Oligonukleotid vorhanden war. Das Fluoreszenzsignal war graduell niedriger, je mehr RNA in der Probe vorhanden war.
Dieser Versuch zeigt, dass das erfindungsgemäße Verfahren geeignet ist, spezifisch Poly(A)RNA und mRNA nachzuweisen und dass nur einzelsträngiges Poly(dT)-Oligonukleotid, das nicht hybridisiert ist, durch die Exonuklease I abgebaut wird, nicht aber Poly(dT)-Oligonukleotide, die hybridisiert in einem Doppelstrang vorliegen. Der Versuch zeigt ebenfalls, dass während der ersten 15 min des Exonuklease I Verdaus der Verlauf der Kurven bei den unterschiedlichen eingesetzten RNA-Mengen parallel verlief und dass bereits nach 1 min Reaktionszeit ein Unterschied zwischen den Fluoreszenzsignalen in Abhängigkeit der eingesetzten RNA-Menge messbar war.

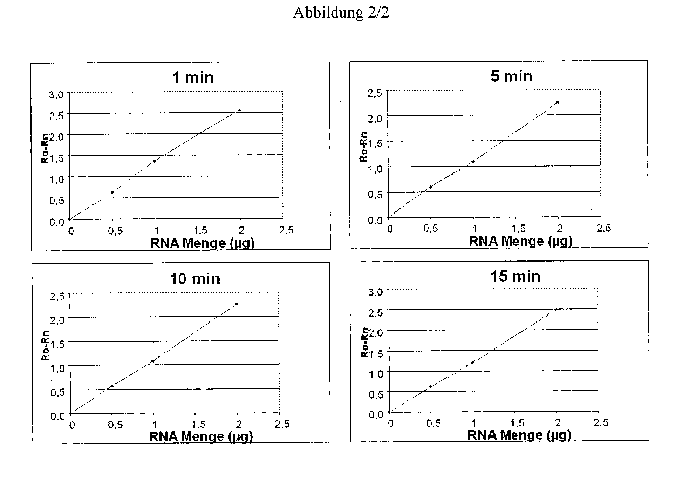

Abbildung 2 zeigt, dass bei allen gemessenen Zeitpunkten (1 min, 5 min, 10 min, 15 min) ein lineares Verhältnis zwischen der eingesetzten RNA-Menge (in µg) und der gemessenen Fluoreszenz besteht. Dies bedeutet, dass sich mit Hilfe einer Standardreihe die Menge an mRNA in einer unbekannten Probe zuverlässig quantifizieren lässt.
Da der Verlauf der Geraden bei den unterschiedlichen gemessenen Zeitpunkten nahezu identische Werte in der Differenz zwischen dem Fluoreszenzsignal ohne eingesetzter RNA und dem Fluoreszenzsignal bei der jeweiligen Probe mit eingesetzter RNA ergab, ist auch hier gezeigt, dass der Reaktionsverlauf zumindest während der ersten 15 min linear ist und sich keine Unterschiede in der Reaktionsgeschwindigkeit bei den eingesetzten RNA-Mengen ergeben und sich somit das erfindungsgemäße Verfahren für die Quantifizierung von RNA-Mengen in einem weiten Konzentrationsbereich eignet.

## Patentansprüche

1. Verfahren zum spezifischen Nachweis und/oder zur Quantifizierung von Poly(A)RNA und/oder mRNA, aufweisend die folgenden Verfahrensschritte:
i) In Kontakt Bringen einer Poly(A)RNA- und/oder mRNA-haltigen Probe mit einem Poly(dT)-Oligonukleotid, welches einen Fluoreszenzfarbstoff sowie einen Quencher aufweist, wobei der Quencher bei der emittierenden Wellenlänge des Fluoreszenzfarbstoffes die Fluoreszenz im Poly(dT)-Oligonukleotid sowohl im hybridisierten als auch im nicht hybridisierten Zustand vollständig löscht;
ii) Hybridisieren des Poly(dT)-Oligonukleotids an die Poly(A)RNA und/oder mRNA, wobei das Fluoreszenz-Signal unverändert bleibt;
iii) In Kontakt Bringen des Hybridisierungsansatzes aus Schritt ii) mit einer Einzelstrang-spezifischen Nuklease, wobei Fluoreszenz-markierte Nukleotide aus denjenigen Poly(di)-Oligonukleotiden freigesetzt werden, die nicht mit Poly(A)RNA und/oder mRNA hybridisiert sind, wodurch das Fluoreszenzsignal erhöht wird;
iv) Messen des entstandenen Fluoreszenzsignals.

2. Verfahren gemäß Anspruch 1, **dadurch gekennzeichnet, dass** das Poly(dT)-Oligonukleotid ausschließlich aus (dT)-Nukleotiden aufgebaut ist

3. Verfahren gemäß Anspruch 1, **dadurch gekennzeichnet, dass** das Poly(dT)-Oligonukleotid eine Mischung aus unterschiedlichen Poly(dT) enthaltenden Oligonukleotiden ist, die an ihrem 3'-Ende mindestens ein weiteres Nukleotid aufweisen, welches nicht ein (dT)-Nukleotid ist.

4. Verfahren gemäß einem der Ansprüche 1 bis 3, **dadurch gekennzeichnet, dass** das Poly(dT)-Oligonukleotid eine Länge von 10 bis 100 Nukleotiden aufweist.

5. Verfahren gemäß Anspruch 4, **dadurch gekennzeichnet, dass** das Poly(dT)-Oligonukleotid eine Länge von 18 bis 25 Nukleotiden aufweist.

6. Verfahren gemäß einem der Ansprüche 1 bis 4, **dadurch gekennzeichnet, dass** der Abstand zwischen Fluoreszenzfarbstoff und Quencher im Poly(dT)-Oligonukleotid maximal 30 Nukleotide beträgt.

7. Verfahren gemäß einem der Ansprüche 1 bis 6, **dadurch gekennzeichnet, dass** Fluoreszenzfarbstoff und Quencher im Poly(dT)-Oligonukleotid am gegenüberliegenden Ende des Oligonukleotids angebracht sind.

8. Verfahren gemäß einem der Ansprüche 1 bis 7, **dadurch gekennzeichnet, dass** der Fluoreszenzfarbstoff ausgewählt aus der Gruppe 6-FAM, 6-JOE, Alexa Fluor 568, Alexa Fluor 633, Alexa Fluor 680, Bodipy, CAL Fluor, CAL Fluor Red 610, TAMRA, HEX, Oregon Green, TET, Texas Red, Marina Blue, Edans Bothell Blue, Fluorescein, Yakima Yellow, Glod 540, Cy3.5 und Cy5 ist

9. Verfahren gemäß einem der Ansprüche 1 bis 7, **dadurch gekennzeichnet, dass** der Quencher ausgewählt aus der Gruppe DABCYL, BHQ1, BHQ2 und TAMRA ist.

10. Verfahren gemäß Anspruch 1, **dadurch gekennzeichnet, dass** die Einzelstrang-spezifische Nuklease eine Exonuklease ist.

11. Verfahren gemäß Anspruch 10, **dadurch gekennzeichnet, dass** die Einzelstrang-spezifische Nuklease ausgewählt aus der Gruppe Exonuklease I, Exonuklease T und RecJ ist oder ein Gemisch von diesen ist.

12. Verfahren gemäß Anspruch 11, **dadurch gekennzeichnet, dass** die Einzelstrangspezifische Exonuklease Exonuklease I oder Exonuklease T ist oder ein Gemisch von diesen ist.

13. Verfahren gemäß Anspruch 1, **dadurch gekennzeichnet, dass** die Einzelstrangspezifische Nuklease eine Endonuklease ist.

14. Verfahren gemäß Anspruch 13, **dadurch gekennzeichnet, dass** die Einzelstrangspezifische Endonuklease S1 Nuklease oder Mung Bean Nuclease oder ein Gemisch von diesen ist.

## Claims

1. Method for specifically detecting and/or for quantifying poly(A) RNA and/or mRNA, comprising the following procedural steps:
i) contacting a poly(A)-RNA-containing sample and/or mRNA-containing sample with a poly(dT) oligonucleotide which features a fluorescent dye and also a quencher, with the quencher, at the emitting wavelength of the fluorescent dye, significantly quenching the fluorescence in the poly(dT) oligonucleotide both in the hybridized state and in the non-hybridized state to the same extent;
ii) hybridizing the poly(dT) oligonucleotide to the poly(A) RNA and/or mRNA, with the fluorescent signal remaining unchanged;
iii) contacting the hybridization mix from step ii) with a single-strand-specific nuclease, with fluorescently labelled nucleotides being released from those poly(dT) oligonucleotides which have not hybridized with poly(A) RNA and/or mRNA, resulting in an increase in the fluorescent signal;
iv) measuring the resulting fluorescent signal.

2. Method according to Claim 1, **characterized in that** the poly(dT) oligonucleotide is composed only of (dT) nucleotides.

3. Method according to Claim 1, **characterized in that** the poly(dT) oligonucleotide is a mixture of different poly(dT)-containing oligonucleotides which feature at their 3' end at least one further nucleotide which is not a (dT) nucleotide.

4. Method according to any one of Claims 1 to 3, **characterized in that** the poly(dT) oligonucleotide has a length in the range from 10 to 100 nucleotides.

5. Method according to Claim 4, **characterized in that** the poly(dT) oligonucleotide has a length in the range from 18 to 25 nucleotides.

6. Method according to any one of Claims 1 to 4, **characterized in that** the distance between the fluorescent dye and the quencher in the poly(dT) oligonucleotide is a maximum of 30 nucleotides.

7. Method according to any one of Claims 1 to 6, **characterized in that** the fluorescent dye and the quencher in the poly(dT) oligonucleotide are attached to the opposite ends of the oligonucleotide.

8. Method according to any one of Claims 1 to 7, **characterized in that** the fluorescent dye is selected from the group comprising 6-FAM, 6-JOE, Alexa Fluor 568, Alexa Fluor 633, Alexa Fluor 680, Bodipy, CAL Fluor, CAL Fluor Red 610, TAMRA, HEX, Oregon Green, TET, Texas Red, Marina Blue, Edans Bothell Blue, Fluorescein, Yakima Yellow, Glod 540, Cy3.5 and Cy5.

9. Method according to any one of Claims 1 to 7, **characterized in that** the quencher is selected from the group comprising DABCYL, BHQ1, BHQ2 and TAMRA.

10. Method according to Claim 1, **characterized in that** the single-strand-specific nuclease is an exonuclease.

11. Method according to Claim 10, **characterized in that** the single-strand-specific nuclease is selected from the group comprising exonuclease I, exonuclease T and RecJ or is a mixture of these nucleases.

12. Method according to Claim 11, **characterized in that** the single-strand-specific exonuclease is exonuclease I or exonuclease T or is a mixture of these exonucleases.

13. Method according to Claim 1, **characterized in that** the single-strand-specific nuclease is an endonuclease.

14. Method according to Claim 13, **characterized in that** the single-strand-specific endonuclease is S1 nuclease or mung bean nuclease or a mixture of these endonucleases.

## Revendications

1. Procédé de détection spécifique et/ou de quantification d'ARN poly(A) et/ou d'ARNm, comprenant les étapes suivantes :
i) mise en contact d'un échantillon contenant de l'ARN poly(A) et/ou de l'ARNm avec un oligonucléotide poly(dT), qui comprend un colorant fluorescent et un extincteur, l'extincteur éteignant, à la longueur d'onde d'émission du colorant fluorescent, la fluorescence de l'oligonucléotide poly(dT), tant à l'état hybridé qu'à l'état non hybridé, d'une manière significative et dans une mesure identique ;
ii) hybridation de l'oligonucléotide poly(dT) à l'ARN poly(A) et/ou à l'ARNm, ce à l'occasion de quoi le signal fluorescent reste inchangé ;
iii) mise en contact de la masse d'hybridation de l'étape ii) avec une nucléase spécifique de simple brin, les nucléotides marqués par fluorescence étant libérés des oligonucléotides poly(dT) qui ne sont pas hybridés à l'ARN poly(A) et/ou à l'ARNm, ce qui augmente le signal de fluorescence ;
iv) mesure du signal de fluorescence produit.

2. Procédé selon la revendication 1, **caractérisé en ce que** l'oligonucléotide poly(dT) est exclusivement constitué de nucléotides (dT).

3. Procédé selon la revendication 1, **caractérisé en ce que** l'oligonucléotide poly(dT) est un mélange de différents oligonucléotides contenant du poly(dT), qui en leur extrémité 3' comportent au moins un autre nucléotide, qui n'est pas un nucléotide (dT).

4. Procédé selon l'une des revendications 1 à 3, **caractérisé en ce que** l'oligonucléotide poly(dT) a une longueur de 10 à 100 nucléotides.

5. Procédé selon la revendication 4, **caractérisé en ce que** l'oligonucléotide poly(dT) a une longueur de 18 à 25 nucléotides.

6. Procédé selon l'une des revendications 1 à 4, **caractérisé en ce que** la distance entre le colorant fluorescent et l'extincteur est dans l'oligonucléotide poly(dT) au maximum de 30 nucléotides.

7. Procédé selon l'une des revendications 1 à 6, **caractérisé en ce que** le colorant fluorescent et l'extincteur sont, dans l'oligonucléotide poly(dT), rapportés à l'extrémité opposée de l'oligonucléotide.

8. Procédé selon l'une des revendications 1 à 7, **caractérisé en ce que** le colorant fluorescent est choisi dans le groupe consistant en 6-FAM, 6-JOE, Alexa Fluor 568, Alexa Fluor 633, Alexa Fluor 680, Bodipy, CAL Fluor, CAL Fluor Red 610, TAMRA, HEX, Oregon Green, TET, Texas Red, Marina Blue, Edans Bothell Blue, fluorescéine, Yakima Yellow, Glod 540, Cy3.5 et Cy5.

9. Procédé selon l'une des revendications 1 à 7, **caractérisé en ce que** l'extincteur est choisi dans le groupe DABCYL, BHQ1, BHQ2 et TAMRA.

10. Procédé selon la revendication 1, **caractérisé en ce que** l'endonucléase spécifique de simple brin est une exonucléase.

11. Procédé selon la revendication 10, **caractérisé en ce que** la nucléase spécifique de simple brin est choisie dans le groupe consistant en l'Exonucléase 1, l'Exonucléase T et RecJ, ou un mélange de ceux-ci.

12. Procédé selon la revendication 11, **caractérisé en ce que** l'exonucléase spécifique de simple brin est l'Exonucléase 1 ou l'Exonucléase T ou un mélange de celles-ci.

13. Procédé selon la revendication 1, **caractérisé en ce que** la nucléase spécifique de simple brin est une endonucléase.

14. Procédé selon la revendication 13, **caractérisé en ce que** l'endonucléase spécifique de simple brin est la nucléase S1 ou la nucléase du haricot mungo, ou un mélange de celles-ci.
